# EUROPEAN PATENT APPLICATION

(11) **EP 1 715 049 A1**
(43) Date of publication of application: **25.10.2006**
(21) Application number: 06014725.3
(22) Date of filing: 30.05.2001
(51) Int. Cl.: C12N 15/53, C12N 9/02

(54) **Method of detecting and quantifying human P450 molecular species and probe and kit for this method**

(30) Priority: 01.06.2000 JP 2000164214
(62) Divisional of application: 01934418.3
(71) Applicant: OTSUKA PHARMACEUTICAL FACTORY, INC., Naruto-shi, Tokushima 772-8601 (JP)
(72) Inventor: Nishimura, Masuhiro, Tokushima-shi Tokushima-ken (JP); Yaguchi, Hiroshi, Naruto-shi Tokushima-ken (JP); Naito, Shinsaku, Tokushima-shi Tokushima-ken (JP); Hiraoka, Isao, Tokushima-shi Tokushima-ken (JP)
(74) Representative: HOFFMANN EITLE

(57) **Abstract**

The present invention provides a kit for detecting and quantifying the CYP3A4 gene of the human cytochrome P450 molecular species, comprising a set of a primer pair of a forward primer comprising an oligonucleotide which hybridizes with the 825-854 region of the CYP3A4 gene and a reverse primer comprising an oligonucleotide which hybridizes with the 973-948 region of the CYP3A4 gene, and a probe comprising an oligonucleotide which hybridizes with the 946-918 region of the CYP3A4 gene, and a method of detecting and quantifying said human cytochrome P 450 molecular species using the kit.

SEQUENCE LISTING

<110> Otsuka Pharmaceutical Factory Inc.

<120> A method for detecting human P450 molecular species and a prove

and a kit therefor

<130> P01-38

<150> 2000-164214

<151> 2000-06-01

<160> 105

<170> PatentIn Ver. 2.0

<210> 1

<211> 26

<212> DNA

<213> human P450 CYP1A1 gene

<400> 1

cgctatgacc acaaccacca agaact 26

<210> 2

<211> 26

<212> DNA

<213> human P450 CYP1A2 gene

<400> 2

ctagagccag cggcaacctc atccca 26

<210> 3

<211> 28

<212> DNA

<213> human P450 CYP1B1 gene

<400> 3
cagctttgtg cctgtcacta ttcctcat 28

<210> 4
<211> 24
<212> DNA
<213> human P450 CYP2A6/7 gene
<400> 4
tggccccgtg ttcaccattc actt 24

<210> 5
<211> 30
<212> DNA
<213> human P450 CYP2A6 gene
<400> 5
tgcctgactg tgatggtctt gatgtctgtt 30

<210> 6
<211> 25
<212> DNA
<213> human P450 CYP2A7 gene
<400> 6
aacctcttca ttgcaggcac cgaga 25

<210> 7
<211> 28
<212> DNA
<213> human P450 CYP2B6 gene
<400> 7

tcatctgctc catcgtcttt ggaaaacg 28

<210> 8
<211> 26
<212> DNA
<213> human P450 CYP2C8 gene
<400> 8
tttctccctc acaaccttgc ggaatt 26

<210> 9
<211> 26
<212> DNA
<213> human P450 CYP2C9 gene
<400> 9
ttgtgcttgt cgtctctgtc ccagct 26

<210> 10
<211> 25
<212> DNA
<213> human P450 CYP2C18 gene
<400> 10
aatgcatttg gtcgtgtgcc accct 25

<210> 11
<211> 27
<212> DNA
<213> human P450 CYP2C19 gene
<400> 11
ttgtgcttgt tgtctctgtc ccagctc 27

<210> 12
<211> 28
<212> DNA
<213> human P450 CYP2D6 gene
<400> 12
cagctggatg agctgctaac tgagcaca 28

<210> 13
<211> 22
<212> DNA
<213> human P450 CYP2E1 gene
<400> 13
tccaacctgc cccatgaagc aa 22

<210> 14
<211> 28
<212> DNA
<213> human P450 CYP2F1 gene
<400> 14
atgacaactt ccaaatcatg agcagccc 28

<210> 15
<211> 24
<212> DNA
<213> human P450 CYP2J2 gene
<400> 15
aggcccaaca cctcactgaa gcaa 24

<210> 16
<211> 30
<212> DNA
<213> human P450 CYP3A3/4 gene
<400> 16
tctggagctc gtggcccaat caattatctt 30

<210> 17
<211> 29
<212> DNA
<213> human P450 CYP3A4 gene
<400> 17
aggagagaac actgctcgtg gtttcacag 29

<210> 18
<211> 29
<212> DNA
<213> human P450 CYP3A5 gene
<400> 18
tttctttcga attctgggag tcaatcatc 29

<210> 19
<211> 32
<212> DNA
<213> human P450 CYP3A7 gene
<400> 19
agtcttttga attctgagag tcaatcatca gc 32

<210> 20

<211> 26
<212> DNA
<213> human P450 CYP4A11 gene
<400> 20
tgacctgaac aacctggttt tttccc 26

<210> 21
<211> 28
<212> DNA
<213> human P450 CYP4B1 gene
<400> 21
ctgtaccctg agcaccagca tcgttgta 28

<210> 22
<211> 24
<212> DNA
<213> human P450 CYP4F2 gene
<400> 22
agtcccggtc atctcccgcc atgt 24

<210> 23
<211> 25
<212> DNA
<213> human P450 CYP4F3 gene
<400> 23
tgccgtctct cgctgctgca cccaa 25

<210> 24
<211> 30

<212> DNA
<213> human P450 CYP4F8 gene
<400> 24
cgaaaacgcc cagaagaggt cacctatggc 30

<210> 25
<211> 26
<212> DNA
<213> human P450 CYP11B1 gene
<400> 25
gcgcgtgttc ctctactctc tgggtc 26

<210> 26
<211> 31
<212> DNA
<213> human P450 CYP11B2 gene
<400> 26
acaggttttc ctctactcgc tgggtcgcaa t 31

<210> 27
<211> 26
<212> DNA
<213> human P450 CYP17 gene
<400> 27
tcagttcatg cctgagcgtt tcttga 26

<210> 28
<211> 29
<212> DNA

<213> human P450 CYP19 gene
<400> 28
ccttctttat gaaagctctg tcaggcccc 29

<210> 29
<211> 28
<212> DNA
<213> human P450 CYP27 gene
<400> 29
tgcgcttctt ctttcagctg ttcgttca 28

<210> 30
<211> 28
<212> DNA
<213> human P450 CYP1B1 gene
<400> 30
agcagctcaa ccgcaacttc agcaactt 28

<210> 31
<211> 28
<212> DNA
<213> human P450 CYP2B6 gene
<400> 31
tgagcactgc tctccatgac ccacacta 28

<210> 32
<211> 32
<212> DNA
<213> human P450 CYP2C8 gene

<400> 32
ttggcactgt agctgatcta tttgttgctg ga                                    32

<210> 33
<211> 28
<212> DNA
<213> human P450 CYP2C9 gene
<400> 33
aaaacactgc agttgacttg tttggagc                                         28

<210> 34
<211> 32
<212> DNA
<213> human P450 CYP2C19 gene
<400> 34
taatcactgc agctgactta cttggagctg gg                                    32

<210> 35
<211> 26
<212> DNA
<213> human P450 CYP4C11 gene
<400> 35
acattcccaa gtgcctgtcc tcattg                                          26

<210> 36
<211> 22
<212> DNA
<213> human P450 CYP1A1 gene
<400> 36

gtcatctgtg ccatttgctt tg                                                22

<210> 37
<211> 22
<212> DNA
<213> human P450 CYP1A1 gene
<400> 37
caaccacctc cccgaaatta tt                                                22

<210> 38
<211> 21
<212> DNA
<213> human P450 CYP1A2 gene
<400> 38
tgttcaagca cagcaagaag g                                                 21

<210> 39
<211> 21
<212> DNA
<213> human P450 CYP1A2 gene
<400> 39
tgctccaaag acgtcattga c                                                 21

<210> 40
<211> 20
<212> DNA
<213> human P450 CYP1B1 gene
<400> 40
ttatgaagcc atgcgcttct                                                   20

<210> 41
<211> 22
<212> DNA
<213> human P450 CYP1B1 gene
<400> 41
agacagaggt gttggcagtg gt 22

<210> 42
<211> 21
<212> DNA
<213> human P450 CYP2A6/7 gene
<400> 42
tcatgaagat cagtgagcgc t 21

<210> 43
<211> 19
<212> DNA
<213> human P450 CYP2A6/7 gene
<400> 43
tcatgtccac acagcacca 19

<210> 44
<211> 19
<212> DNA
<213> human P450 CYP2A6 gene
<400> 44
ttttggtggc cttgctggt 19

<210> 45
<211> 26
<212> DNA
<213> human P450 CYP2A6 gene
<400> 45
ggagttgtac atctgctctg tgttca 26

<210> 46
<211> 24
<212> DNA
<213> human P450 CYP2A7 gene
<400> 46
cttgaagaac ctgatgatga gcac 24

<210> 47
<211> 23
<212> DNA
<213> human P450 CYP2A7 gene
<400> 47
ctctgtcaat ctcctcatgg acc 23

<210> 48
<211> 19
<212> DNA
<213> human P450 CYP2B6 gene
<400> 48
ccattccatt accgccaac 19

<210> 49

<211> 24

<212> DNA

<213> human P450 CYP2B6 gene

<400> 49

aggaactctt gatcttggta gtgg 24

<210> 50

<211> 21

<212> DNA

<213> human P450 CYP2C8 gene

<400> 50

aagagatgga aggagatccg g 21

<210> 51

<211> 19

<212> DNA

<213> human P450 CYP2C8 gene

<400> 51

tcaatgctcc tcttcccca 19

<210> 52

<211> 24

<212> DNA

<213> human P450 CYP2C9 gene

<400> 52

ctcctatcat tgattacttc ccgg 24

<210> 53

<211> 23

<212> DNA
<213> human P450 CYP2C9 gene
<400> 53
ggagaaggag agcatatctc agg 23

<210> 54
<211> 21
<212> DNA
<213> human P450 CYP2C18 gene
<400> 54
aggatattga catcaccccc a 21

<210> 55
<211> 23
<212> DNA
<213> human P450 CYP2C18 gene
<400> 55
tcagacagga atgaagcaga gct 23

<210> 56
<211> 23
<212> DNA
<213> human P450 CYP2C19 gene
<400> 56
cttggtaatc actgcagctg act 23

<210> 57
<211> 23
<212> DNA

<213> human P450 CYP2C19 gene

<400> 57

tcagcaggag aaggagagca tat                                                23

<210> 58

<211> 21

<212> DNA

<213> human P450 CYP2D6 genc

<400> 58

cctacgcttc caaaaggctt t                                                  21

<210> 59

<211> 22

<212> DNA

<213> human P450 CYP2D6 gene

<400> 59

agagaacagg tcagccacca ct                                                 22

<210> 60

<211> 19

<212> DNA

<213> human P450 CYP2E1 gene

<400> 60

ttcagcggtt catcaccct                                                     19

<210> 61

<211> 24

<212> DNA

<213> human P450 CYP2E1 gene

<400> 61
gaggtatcct ctgaaaatgg tgtc                                                24

<210> 62
<211> 22
<212> DNA
<213> human P450 CYP2F1 gene
<400> 62
gctcaccatt atccgcctta tc                                                  22

<210> 63
<211> 22
<212> DNA
<213> human P450 CYP2F1 gene
<400> 63
gaggtctctc aggcacttga ag                                                  22

<210> 64
<211> 23
<212> DNA
<213> human P450 CYP2J2 gene
<400> 64
agcttagagg aacgcattca gga                                                 23

<210> 65
<211> 22
<212> DNA
<213> human P450 CYP2J2 gene
<400> 65

cgaaggtgat ggagcaaatg at 22

<210> 66
<211> 23
<212> DNA
<213> human P450 CYP3A3/4 gene
<400> 66
actgagtccc acaaagctct gtc 23

<210> 67
<211> 22
<212> DNA
<213> human P450 CYP3A3/4 gene
<400> 67
aactgcatca atttcctcct gc 22

<210> 68
<211> 30
<212> DNA
<213> human P450 CYP3A4 gene
<400> 68
gattgactct cagaattcaa aagaaactga 30

<210> 69
<211> 26
<212> DNA
<213> human P450 CYP3A4 gene
<400> 69
ggtgagtggc cagttcatac ataatg 26

<210> 70
<211> 22
<212> DNA
<213> human P450 CYP3A5 gene
<400> 70
ccttacccca gtttttgaag ca                                                22

<210> 71
<211> 23
<212> DNA
<213> human P450 CYP3A5 gene
<400> 71
tccagatcag acagagcttt gtg                                               23

<210> 72
<211> 22
<212> DNA
<213> human P450 CYP3A7 gene
<400> 72
ccttacccca attcttgaag ca                                                22

<210> 73
<211> 23
<212> DNA
<213> human P450 CYP3A7 gene
<400> 73
tccagatcag acagagcttt gtg                                               23

<210> 74
<211> 24
<212> DNA
<213> human P450 CYP4A11 gene
<400> 74
tctcagtcct acatacaggc catt 24

<210> 75
<211> 22
<212> DNA
<213> human P450 CYP4A11 gene
<400> 75
gttggatcac ttggtctgtg tg 22

<210> 76
<211> 22
<212> DNA
<213> human P450 CYP4B1 gene
<400> 76
cctggtttct ctactgcatg gc 22

<210> 77
<211> 21
<212> DNA
<213> human P450 CYP4B1 gene
<400> 77
ccagatcatc ccactggaag a 21

<210> 78

<211> 22

<212> DNA

<213> human P450 CYP4F2 gene

<400> 78

ccgtgagcct aaagagattg aa                                                22

<210> 79

<211> 21

<212> DNA

<213> human P450 CYP4F2 gene

<400> 79

cgaaaacact gatgaggcag a                                                 21

<210> 80

<211> 22

<212> DNA

<213> human P450 CYP4F3 gene

<400> 80

tacaagagct tctgaaggac cg                                                22

<210> 81

<211> 22

<212> DNA

<213> human P450 CYP4F3 gene

<400> 81

tgatgaggca gataatgcct tt                                                22

<210> 82

<211> 22

<212> DNA
<213> human P450 CYP4F8 gene
<400> 82
aatccatcac aacccctcag tc                                                22

<210> 83
<211> 20
<212> DNA
<213> human P450 CYP4F8 gene
<400> 83
ccgccgagaa aggaataaaa                                                   20

<210> 84
<211> 22
<212> DNA
<213> human P450 CYP11B1 gene
<400> 84
gagctcagac ttggtgcttc ag                                                22

<210> 85
<211> 20
<212> DNA
<213> human P450 CYP11B1 gene
<400> 85
tggaggtgtt tcagcacatg                                                   20

<210> 86
<211> 23
<212> DNA

<213> human P450 CYP11B2 gene
<400> 86
cttggtgctt cagaactacc aca 23

<210> 87
<211> 23
<212> DNA
<213> human P450 CYP11B2 gene
<400> 87
ttagtgtctc caccaggaag tgc 23

<210> 88
<211> 22
<212> DNA
<213> human P450 CYP17 gene
<400> 88
tcacaatgag aaggagtggc ac 22

<210> 89
<211> 23
<212> DNA
<213> human P450 CYP17 gene
<400> 89
agcttactga cggtgagatg agc 23

<210> 90
<211> 22
<212> DNA
<213> human P450 CYP19 gene

<400> 90
agagctctgg aaaacaactc ga                                                22

<210> 91
<211> 21
<212> DNA
<213> human P450 CYP19 gene
<400> 91
ctgtgaccat acgaacaagg c                                                 21

<210> 92
<211> 23
<212> DNA
<213> human P450 CYP27 gene
<400> 92
agaggagatt ccacgtctag gac                                               23

<210> 93
<211> 22
<212> DNA
<213> human P450 CYP27 gene
<400> 93
acatccacat tggaccgtac tt                                                22

<210> 94
<211> 18
<212> DNA
<213> human P450 CYP1B1 gene
<400> 94

accgttttcc gcgaattc 18

<210> 95
<211> 21
<212> DNA
<213> human P450 CYP1B1 gene
<400> 95
gtacgttctc caaatccagc c 21

<210> 96
<211> 23
<212> DNA
<213> human P450 CYP2B6 gene
<400> 96
ccccaaggac acagaagtat ttc 23

<210> 97
<211> 22
<212> DNA
<213> human P450 CYP2B6 gene
<400> 97
gattgaaggc gtctggtttt tc 22

<210> 98
<211> 22
<212> DNA
<213> human P450 CYP2C8 gene
<400> 98
ggactttatc gattgcttcc tg 22

<210> 99
<211> 22
<212> DNA
<213> human P450 CYP2C8 gene
<400> 99
ccatatctca gagtggtgct tg 22

<210> 100
<211> 24
<212> DNA
<213> human P450 CYP2C9 gene
<400> 100
gacatgaaca accctcagga cttt 24

<210> 101
<211> 20
<212> DNA
<213> human P450 CYP2C9 gene
<400> 101
tgcttgtcgt ctctgtccca 20

<210> 102
<211> 22
<212> DNA
<213> human P450 CYP2C19 gene
<400> 102
gaacaccaag aatcgatgga ca 22

<210> 103
<211> 22
<212> DNA
<213> human P450 CYP2C19 gene
<400> 103
tcagcaggag aaggagagca ta 22

<210> 104
<211> 22
<212> DNA
<213> human P450 CYP4A11 gene
<400> 104
aggagctaca acggattcag aa 22

<210> 105
<211> 21
<212> DNA
<213> human P450 CYP4A11 gene
<400> 105
acgaactttg cctccccata g 21

## Description

### TECHNICAL FIELD

The present invention relates to a method of detecting and quantifying human P450 molecular species, and probes and a kit for use in the method.

### BACKGROUND OF THE INVENTION

Cytochrome P450 is one of the enzymes that participate in the metabolism of chemical substances, such as active ingredient compounds in medicines. A number of molecular species of cytochrome P450 are known to exist, and about 30 molecular species have been hitherto confirmed in humans. Although these molecular species are different in enzyme activity from one another, they are each reported to participate in the metabolism of active ingredient compounds in medicines (e.g., active ingredient compounds in tricyclic antidepressants, antiepileptics, benzodiazepine preparations, β-blockers, barbital sleep-inducing hypnotics, and other medicines, dimethylnitrosamine, etc.), benzene and other organic solvents, low-molecular-weight carcinogens in the environment, or the like (for example, Yoo, J.S.H., Chung, R., C., Wade, D., & Yang, C.S.(1987) Cancer Res., 47, 3378-3383).

In the development of a new medicine, it is important to understand the actions (changes in expression levels) of molecular species of human cytochrome P450 (hereinafter referred to simply as "P450") upon administration of the new medicine. This is because, if the changes in the expression levels of P450 molecular species are unknown, it is impossible to predict an increase or decrease in efficacy and side effects of the new medicine caused by a medicine or another substance concurrently administered with the new medicine, or an increase or decrease in efficacy and side effects of the medicine concurrently administered. Thus, the safety of the new medicine cannot be confirmed.

Therefore, in pharmaceutical fields, such as new medicine development, information concerning P450 molecular species, and in particular, a technique for measuring the levels of P450 molecular species individually to obtain information concerning each P450 molecular species, have been desired. The development of a technique for measuring the molecular species individually would make it possible to, for example, easily understand the interaction of a new medicine with another medicine and the influence of the new medicine on organisms in a special morbid condition, and to acquire information about the side effects of the new medicine, thereby ensuring its safety.

The polymerase chain reaction (PCR) is a widely known method for amplifying nucleic acids. Of the PCR techniques, RT-PCR (Reverse Transcription-PCR), competitive RT-PCR and the like are used for detecting and quantifying a trace amount of mRNA, and show their effectiveness.

In recent years, a real-time quantitative detection technique using PCR has been established (TaqMan PCR, Genome Res., 6 (10), 986 (1996), ABI PRISM^{™} Sequence Detection System, Applied Biosystems). This technique measures the amount of nucleic acids using a particular fluorescent-labeled probe (TaqMan probe). More specifically, this technique utilizes the following principles: For example, a fluorescent-labeled probe having a reporter dye at the 5' end and a quencher dye at the 3' end is annealed to the target DNA, and the DNA is subjected to normal PCR. As the extension reaction proceeds, the probe is hydrolyzed from the 5' end by the 5'-3' exonuclease activity possessed by DNA polymerase. As a result, the reporter dye at the 5' end is separated from the quencher dye at the 3' end, thereby eliminating the FRET (Fluorescence Resonance Energy Transfer, the reduction in fluorescence intensity owing to the decrease in the energy level of the reporter dye caused by the resonance of the two fluorescent dyes) effect produced by the spatial proximity between the two dyes, and increasing the fluorescence intensity of the reporter dye that has been controlled by the quencher dye. The target nucleic acid can be selectively quantified and detected in real-time by measuring the increase of the fluorescence intensity.

This technique is advantageous in that it can test various samples simultaneously in a short time, since, unlike the detection and quantification technique using conventional PCR it does not involve complicated steps, such as agarose gel electrophoresis of the amplified product after PCR and analysis of the electrophoresis pattern.

Generally, when conducting clinical tests in a clinical test center or the like, it is necessary to inspect an extremely large number of samples within a limited time. Therefore, there is considerable demand for the development of efficient test techniques. The real-time quantitative detection technique is a promising candidate to meet this demand.

The present inventors turned their attention to the real-time quantitative detection technique using PCR, and conceived that, if the detection technique can be utilized for detecting human P450 molecular species, the molecular species can be individually detected and quantified using the same apparatus under the same PCR conditions.

However, as described above, there are about 30 presently known P450 molecular species participating in the metabolism of chemical substances. Although the mRNA sequence of each species is known, it was deemed to be difficult to find oligonucleotides (for use as primer pairs) that do not overlap with one another and are capable of sufficiently amplifying these molecular species as target genes and hybridizing with particular portions of the target genes. It has been also assumed difficult to constitute, in a specific region between the primer pair, a probe that is capable of hybridizing with the target gene faster than the primers under the same PCR conditions, and is specific to only one molecular species. In particular, it is known that, although the ease of hybridization between two nucleic acids of known nucleotide sequences can be estimated to a certain extent by calculating the melting point (Tm), the combination of primers and a probe selected based on the estimation does not necessarily bring good results in DNA measurement. Therefore, many trial-and-error experiments by experts are expected to be required to select, for each of all the presently known P450 molecular species, a combination of a primer pair and probe for real-time PCR detection which is capable of measuring its gene individually.

### DISCLOSURE OF THE INVENTION

An object of the present invention is to provide a method of detecting and quantifying the genes encoding P450 molecular species, and especially a method of detecting and quantifying the genes by the real-time quantitative detection technique.

Another object of the present invention is to provide probes and primer pairs for use in the above method.

In order to achieve the above objects, the present inventors repeatedly conducted a great number of experiments and research on P450 molecular species. As a result, the present inventors succeeded in finding sets consisting of a primer pair and a probe that are specific to 29 particular genes of P450 molecular species, i.e., sets of a primer pair and a probe that are capable of individually detecting and quantifying the genes encoding the molecular species. The present invention has been accomplished based on this finding.

The present invention provides probes for use in detecting and quantifying P450 molecular species, each of the probes comprising an oligonucleotide hybridizable with one of the following gene regions (1) to (29).
(1) The 616-641 region of the CYP1A1 gene
(2) The 884-909 region of the CYP1A2 gene
(3) The 1176-1203 region or the 785-812 region of the CYP1B1 gene
(4) The 195-218 region of the CYP2A6/7 gene
(5) The 40-69 region of the CYP2A6 gene
(6) The 889-913 region of the CYP2A7 gene
(7) The 533-560 region or the 1175-1202 region of the CYP2B6 gene
(8) The 375-400 region or the 863-894 region of the CYP2C8 gene
(9) The 913-888 region or the 863-890 region of the CYP2C9 gene
(10) The 1420-1444 region of the CYP2C18 gene
(11) The 913-887 region or the 863-894 region of the CYP2C19 gene
(12) The 748-775 region of the CYP2D6 gene
(13) The 1096-1117 region of the CYP2E1 gene
(14) The 608-635 region of the CYP2F1 gene
(15) The 485-508 region of the CYP2J2 gene
(16) The 876-905 region of the CYP3A3/4 gene
(17) The 946-918 region of the CYP3A4 gene
(18) The 850-822 region of the CYP3A5 gene
(19) The 850-819 region of the CYP3A7 gene
(20) The 666-691 region or the 241-266 region of the CYP4A11 gene
(21) The 997-1024 region of the CYP4B1 gene
(22) The 1182-1205 region of the CYP4F2 gene
(23) The 1188-1212 region ot the CYP4F3 gene
(24) The 1341-1370 region of the CYP4F8 gene
(25) The 1209-1234 region of the CYP11B1 gene
(26) The 1209-1239 region of the CYP11B2 gene
(27) The 1230-1255 region of the CYP17 gene
(28) The 437-465 region of the CYP19 gene
(29) The 197-224 region of the CYP27 gene

More specifically, the present invention provides the above probes further comprising a reporter dye and a quencher dye both attached to the oligonucleotide; the above probes in which the oligonucleotide has a base length of 20 to 40; the above probes in which the oligonucleotide comprises a sequence shown in one of SEQ ID NO: 1 to SEQ ID NO: 35; and the above probes in which the oligonucleotide consists of a sequence shown in one of SEQ ID NO: 1 to SEQ ID NO: 35.

Further, the present invention provides a kit for detecting and quantifying one or more P450 molecular species, the kit comprising at least one set of a primer pair of a forward primer and a reverse primer, and a probe, the primers and the probe each comprising an oligonucleotide hybridizable with the gene region shown in (1) to (35) below.
(1) Primer pair: the 589-610 region and the 685-664 region of the CYP1A1 gene; Probe: the 616-641 region of the gene
(2) Primer pair: the 860-880 region and the 951-931 region of the CYP1A2 gene; Probe: the 884-909 region of the gene
(3) Primer pair: the 1155-1174 region and the 1228-1207 region of the CYP1B1 gene; Probe: the 1176-1203 region of the gene
(4) Primer pair: the 173-193 region and the 254-236 region of the CYP2A6/7 gene; Probe: the 195-218 region of the gene
(5) Primer pair: the 20-48 region and the 171-146 region of the CYP2A6 gene; Probe: the 40-69 region of the gene
(6) Primer pair: the 861-884 region and the 1000-978 region of the CYP2A7 gene; Probe: the 889-913 region of the gene
(7) Primer pair: the 513-531 region and the 587-564 region of the CYP2B6 gene; Probe: the 533-560 region of the gene
(8) Primer pair: the 352-372 region and the 425-407 region of the CYP2C8 gene; Probe: the 375-400 region of the gene
(9) Primer pair: the 659-682 region and the 937-915 region of the CYP2C9 gene; Probe: the 913-888 region of the gene
(10) Primer pair: the 1394-1414 region and the 1473-1451 region of the CYP2C18 gene; Probe: the 1420-1444 region of the gene
(11) Primer pair: the 858-880 region and the 943-921 region of the CYP2C19 gene; Probe: the 913-887 region of the gene
(12) Primer pair: the 720-740 region and the 912-891 region of the CYP2D6 gene; Probe: the 748-775 region of the gene
(13) Primer pair: the 1070-1088 region and the 1146-1123 region of the CYP2E1 gene; Probe: the 1096-1117 region of the gene
(14) Primer pair: the 585-606 region and the 744-723 region of the CYP2F1 gene; Probe: the 608-635 region of the gene
(15) Primer pair: the 460-482 region and the 592-571 region of the CYP2J2 gene; Probe: the 485-508 region of the gene
(16) Primer pair: the 850-872 region and the 1014-993 region of the CYP3A3/4 gene; Probe: the 876-905 region of the gene
(17) Primer pair: the 825-854 region and the 973-948 region of the CYP3A4 gene; Probe: the 946-918 region of the gene
(18) Primer pair: the 684-705 region and the 881-859 region of the CYP3A5 gene; Probe: the 850-822 region of the gene
(19) Primer pair: the 684-705 region and the 881-859 region of the CYP3A7 gene; Probe: the 850-819 region of the gene
(20) Primer pair: the 640-663 region and the 805-784 region of the CYP4A11 gene; Probe: the 666-691 region of the gene
(21) Primer pair: the 974-995 region and the 1081-1061 region of the CYP4B1 gene; Probe: the 997-1024 region of the gene
(22) Primer pair: the 1092-1113 region and the 1276-1256 region of the CYP4F2 gene; Probe: the 1182-1205 region of the gene
(23) Primer pair: the 1073-1094 region and the 1267-1246 region of the CYP4F3 gene; Probe: the 1188-1212 region of the gene
(24) Primer pair: the 1278-1299 region and the 1390-1371 region of the CYP4F8 gene; Probe: the 1341-1370 region of the gene
(25) Primer pair: the 1158-1179 region and the 1415-1396 region of the CYP11B1 gene; Probe: the 1209-1234 region of the gene
(26) Primer pair: the 1167-1189 region and the 1429-1407 region of the CYP11B2 gene; Probe: the 1209-1239 region of the gene
(27) Primer pair: the 1200-1221 region and the 1294-1272 region of the CYP17 gene; Probe: the 1230-1255 region of the gene
(28) Primer pair: the 414-435 region and the 487-467 region of the CYP19 gene; Probe: the 437-465 region of the gene
(29) Primer pair: the 171-193 region and the 292-271 region of the CYP27 gene; Probe: the 197-224 region of the gene
(30) Primer pair: the 766-783 region and the 961-941 region of the CYP1B1 gene; Probe: the 785-812 region of the gene
(31) Primer pair: the 1146-1168 region and the 1228-1207 region of the CYP2B6 gene; Probe: the 1175-1202 region of the gene
(32) Primer pair: the 783-804 region and the 926-905 region of the CYP2C8 gene; Probe: the 863-894 region of the gene
(33) Primer pair: the 766-789 region and the 910-891 region of the CYP2C9 gene; Probe: the 863-890 region of the gene
(34) Primer pair: the 748-769 region and the 943-922 region of the CYP2C19 gene; Probe: the 863-894 region of the gene
(35) Primer pair: the 209-230 region and the 288-268 region of the CYP4A11 gene; Probe: the 241-266 region of the gene

More specifically, the present invention provides the above kit in which the probe further comprises a reporter dye and a quencher dye both attached to the oligonucleotide.

Preferably, the set of a primer pair and a probe in the above kit is selected from the following sets (1) to (35), i.e., sets in which the primers and probe each comprise a sequence of the SEQ ID NO shown below. More preferably, the primers and probe in the set each consist of a sequence of the SEQ ID NO shown below.
(1) A set of a primer pair comprising the sequences shown in SEQ ID NOS: 36 and 37 and a probe comprising the sequence shown in SEQ ID NO: 1
(2) A set of a primer pair comprising the sequences shown in SEQ ID NOS: 38 and 39 and a probe comprising the sequence shown in SEQ ID NO: 2
(3) A set of a primer pair comprising the sequences shown in SEQ ID NOS: 40 and 41 and a probe comprising the sequence shown in SEQ ID NO: 3
(4) A set of a primer pair comprising the sequences shown in SEQ ID NOS: 42 and 43 and a probe comprising the sequence shown in SEQ ID NO: 4
(5) A set of a primer pair comprising the sequences shown in SEQ ID NOS: 44 and 45 and a probe comprising the sequence shown in SEQ ID NO: 5
(6) A set of a primer pair comprising the sequences shown in SEQ ID NOS: 46 and 47 and a probe comprising the sequence shown in SEQ ID NO: 6
(7) A set of a primer pair comprising the sequences shown in SEQ ID NOS: 48 and 49 and a probe comprising the sequence shown in SEQ ID NO: 7
(8) A set of a primer pair comprising the sequences shown in SEQ ID NOS: 50 and 51 and a probe comprising the sequence shown in SEQ ID NO: 8
(9) A set of a primer pair comprising the sequences shown in SEQ ID NOS: 52 and 53 and a probe comprising the sequence shown in SEQ ID NO: 9
(10) A set of a primer pair comprising the sequences shown in SEQ ID NOS: 54 and 55 and a probe comprising the sequence shown in SEQ ID NO: 10
(11) A set of a primer pair comprising the sequences shown in SEQ ID NOS: 56 and 57 and a probe comprising the sequence shown in SEQ ID NO: 11
(12) A set of a primer pair comprising the sequences shown in SEQ ID NOS: 58 and 59 and a probe comprising the sequence shown in SEQ ID NO: 12
(13) A set of a primer pair comprising the sequences shown in SEQ ID NOS: 60 and 61 and a probe comprising the sequence shown in SEQ ID NO: 13
(14) A set of a primer pair comprising the sequence shown in SEQ ID NOS: 62 and 63 and a probe comprising the sequence shown in SEQ ID NO: 14
(15) A set of a primer pair comprising the sequences shown in SEQ ID NOS: 64 and 65 and a probe comprising the sequence shown in SEQ ID NO: 15
(16) A set of a primer pair comprising the sequences shown in SEQ ID NOS: 66 and 67 and a probe comprising the sequence shown in SEQ ID NO: 16
(17) A set of a primer pair comprising the sequences shown in SEQ ID NOS: 68 and 69 and a probe comprising the sequence shown in SEQ ID NO: 17
(18) A set of a primer pair comprising the sequences shown in SEQ ID NOS: 70 and 71 and a probe comprising the sequence shown in SEQ ID NO: 18
(19) A set of a primer pair comprising the sequence shown in SEQ ID NOS: 72 and 73 and a probe comprising the sequence shown in SEQ ID NO: 19
(20) A set of a primer pair comprising the sequences shown in SEQ ID NOS: 74 and 75 and a probe comprising the sequence shown in SEQ ID NO: 20
(21) A set of a primer pair comprising the sequence shown in SEQ ID NOS: 76 and 77 and a probe comprising the sequence shown in SEQ ID NO: 21
(22) A set of a primer pair comprising the sequences shown in SEQ ID NOS: 78 and 79 and a probe comprising the sequence shown in SEQ ID NO: 22
(23) A set of a primer pair comprising the sequences shown in SEQ ID NOS: 80 and 81 and a probe comprising the sequence shown in SEQ ID NO: 23
(24) A set of a primer pair comprising the sequences shown in SEQ ID NOS: 82 and 83 and a probe comprising the sequence shown in SEQ ID NO: 24
(25) A set of a primer pair comprising the sequences shown in SEQ ID NOS: 84 and 85 and a probe comprising the sequence shown in SEQ ID NO: 25
(26) A set of a primer pair comprising the sequences shown in SEQ ID NOS: 86 and 87 and a probe comprising the sequence shown in SEQ ID NO: 26
(27) A set of a primer pair comprising the sequences shown in SEQ ID NOS: 88 and 89 and a probe comprising the sequence shown in SEQ ID NO: 27
(28) A set of a primer pair comprising the sequences shown in SEQ ID NOS: 90 and 91 and a probe comprising the sequence shown in SEQ ID NO: 28
(29) A set of a primer pair comprising the sequences shown in SEQ ID NOS: 92 and 93 and a probe comprising the sequence shown in SEQ ID NO: 29
(30) A set of a primer pair comprising the sequence shown in SEQ ID NOS: 94 and 95 and a probe comprising the sequence shown in SEQ ID NO: 30
(31) A set of a primer pair comprising the sequence shown in SEQ ID NOS: 96 and 97 and a probe comprising the sequence shown in SEQ ID NO: 31
(32) A set of a primer pair comprising the sequences shown in SEQ ID NOS: 98 and 99 and a probe comprising the sequence shown in SEQ ID NO: 32
(33) A set of a primer pair comprising the sequences shown in SEQ ID NOS: 100 and 101 and a probe comprising the sequence shown in SEQ ID NO: 33
(34) A set of a primer pair comprising the sequences shown in SEQ ID NOS: 102 and 103 and a probe comprising the sequence shown in SEQ ID NO: 34
(35) A set of a primer pair comprising the sequences shown in SEQ ID NOS: 104 and 105 and a probe comprising the sequence shown in SEQ ID NO: 35

The above sets are used for detecting and quantifying the following P450 molecular species.
Set (1): CYP1A1
Set (2): CYP1A2
Sets (3) and (30): CYP1B1
Set (4): CYP2A6/7
Set (5): CYP2A6
Set (6): CYP2A7
Sets (7) and (31): CYP2B6 Sets (8) and (32): CYP2C8 Sets (9) and (33): CYP2C9 Set (10): CYP2C18
Sets (11) and (34): CYP2C19
Set (12): CYP2D6
Set (13): CYP2E1
Set (14): CYP2F1
Set (15): CYP2J2
Set (16): CYP3A3/4
Set (17): CYP3A4
Set (18): CYP3A5
Set (19): CYP3A7
Sets (20) and (35): CYP4A11
Set (21): CYP4B1
Set (22): CYP4F2
Set (23): CYP4F3
Set (24): CYP4F8
Set (25): CYP11B1
Set (26): CYP11B2
Set (27): CYP17
Set (28): CYP19
Set (29): CYP27

The present invention further provides a method of detecting and quantifying one or more P450 molecular species, the method comprising the steps (a) to (c) described below, in particular, the method wherein, in step (c), the hydrolyzed probe or proves are detected and quantified by detecting the fluorescence produced by irradiation with excitation light, and measuring the degree of fluorescence:
(a) preparing a sample containing a P450 gene or genes;
(b) subjecting the sample to polymerase chain reaction (PCR, which may be RT-PCR) using a kit of the present invention comprising at least one of the above sets of a primer pair and a probe to amplify the gene or genes encoding the P450 molecular species in the sample; and
(c) detecting and measuring the probe or probes hydrolyzed during the polymerase chain reaction.

In this specification, the abbreviations for amino acids, peptides, nucleotide sequences, nucleic acids, etc., are those provided in the IUPAC-IUB Communication on Biological Nomenclature, Eur. J. Biochem., 138: 9 (1984) or "Guideline for preparation of a specification containing nucleotide sequences or amino acid sequences" (JPO), or those conventionally used in this field.

The term "gene" in this specification includes double-stranded DNA and single-stranded sense or antisense DNA constituting double-stranded DNA. The term further includes mRNA involved in the expression of these genes, and cDNA complementary to the mRNA. The term "nucleotide" ("oligonucleotide") includes RNA and DNA.

It is more preferable to perform the method of the present invention according to the real-time detection technique using a probe labeled with a reporter dye and a quencher dye.

The real-time detection technique is capable of easily and rapidly quantifying the genes of molecular species individually in real-time, under the same PCR or RT-PCR conditions, and the establishment of this technique is very beneficial to the clinical and pharmaceutical fields. For example, the technique can easily quantify the P450 mRNA expression in a human sample, which is important in kinetics tests for the development of medicines, thereby making it possible to know the activity of a P450 molecular species when exposed to a chemical substance, such as a medicine. In particular, in the development of new medicines, researchers are required to investigate the activity of the metabolism by P450. Conventionally, the activity of the metabolism by P450 has been investigated by measuring the expression level of each P450 molecular species separately, by a complicated, labor- and time-consuming process. In contrast, the present invention provides a method for easily and rapidly quantifying the expression levels of P450 molecular species under the same conditions, which is extremely useful especially in the development of new medicines.

The method of the present invention is also effective for investigating the mRNA expression of P450 molecular species in an isolated tissue or a tissue obtained by biopsy. Thus, the method of the present invention is advantageous in that it can easily and rapidly process a variety of samples and achieve the desired detection using the total RNA extracted from a small amount of tissue.

There is a possibility that the change in the expression level of a particular P450 molecular species by enzyme induction or other process in the liver, kidney or the like correlates with the change of the expression level in the blood (nuclear cells in blood). However, since the expression level in blood is low, a large amount of blood is required for the measurement. The measurement method of the present invention, which uses any set of the above primer pairs and probes designed by the present inventors, is capable of easily and rapidly detecting and quantifying P450 molecular species using a small amount of sample and common equipment. Therefore, the method of the present invention can detect and quantify the genes encoding each P450 molecular species in blood or a similar sample, thereby making it possible to estimate the expression levels of the molecular species.

### Probes and primers of the present invention

The probes and primers of the present invention each comprise an oligonucleotide hybridizable with a specific region of a molecular species of P450 gene.

As used herein, the term "hybridizable" means being capable of hybridizing with the specific region under the following PCR (RT-PCR) conditions: 1 cycle of 48°C for 30 minutes, 1 cycle of 95°C for 10 minutes, and 50 cycles each consisting of 95°C for 15 seconds and 60°C for 1 minute.

Generally, the hybridizable oligonucleotide has a nucleotide sequence complementary to that of the specific region. However, it is known in this field that even if a primer or probe made of, for example, about 20 nucleotides, has about 1 or 2 mismatches with the template strand, the primer or probe hybridizes with the template strand and thus functions as a PCR primer or a detection probe. Accordingly, the primers and probes of the present invention include those comprising a nucleotide sequence with a small number of mismatches. However, the number of mismatches is preferably as small as possible.

The probes and primers of the present invention are required to have features that: they yield amplification products with a length of about 50 to about 400 bp; that the primers in each set is as close as possible to the probe; that the proportion of G (guanine) and C (cytosine) in each sequence is as close as possible to 50%; and that they do not contain four or more G (guanine) nucleotides in a row. Another feature required of the probes of the present invention is that they have a Tm of about 70°C. Further, the primers are required to have a Tm of about 60°C.

The number of nucleotides in the oligonucleotide for each primer or probe satisfying the above requirements is at least 15, usually 15 to 50, preferably 20 to 40. If the number of nucleotides in the primer or probe is much larger than the above range, the primer or probe is difficult to hybridize with a single-stranded DNA. On the other hand, if the number of nucleotides is too small, the hybridization specificity reduces.

Specific examples of nucleotide sequences for the primers and probes are as described above. The sequences shown in SEQ ID NOS: 1 to 35 (probes) and SEQ ID NOS: 36 to 105 (primers) are used depending on the molecular species.

Preferably, the oligonucleotides for use as the probes and primers of the present invention consist of a nucleotide sequence shown in one of these SEQ ID NOS. However, they are not limited to such oligonucleotides, and may have a small number of mismatches as long as they predominantly comprise one of these nucleotide sequences.

The mRNA sequences of P450 molecular species are known, and are registered in GenBank under the following accession numbers.
(1) The CYP1A1 gene: NM_000499
(2) The CYP1A2 gene: AF182274
(3) The CYP1B1 gene: NM_000104
(4) The CYP2A6/7 gene: AF182275/NM_000764
(5) The CYP2A6 gene: AF182275
(6) The CYP2A7 gene: NM_000764
(7) The CYP2B6 gene: AF182277
(8) The CYP2C8 gene: NM_000770
(9) The CYP2C9 gene: M61857
(10) The CYP2C18 gene: M61856
(11) The CYP2C19 gene: NM_000769
(12) The CYP2D6 gene: NM_000106
(13) The CYP2E1 gene: AF182276
(14) The CYP2F1 gene: NM_000774
(15) The CYP2J2 gene: NM_000775
(16) The CYP3A3/4 gene: NM_000776/AF182273
(17) The CYP3A4 gene: AF182273
(18) The CYP3A5 gene: NM_000777
(19) The CYP3A7 gene: NM_000765
(20) The CYP4A11 gene: NM_000778
(21) The CYP4B1 gene: NM_000779
(22) The CYP4F2 gene: NM_001082
(23) The CYP4F3 gene: NM_000896
(24) The CYP4F8 gene: NM_007253
(25) The CYP11B1 gene: NM_000497
(26) The CYP11B2 gene: NM_000498
(27) The CYP17 gene: NM_000102
(28) The CYP19 gene: NM_000103
(29) The CYP27 gene: M62401

In this specification, the specific regions of the molecular species are indicated by the gene sequences registered under the above accession numbers.

The oligonucleotides for use as the probes and primers of the present invention can be easily synthesized in a routine manner, using an automatic synthesizer, such as a DNA synthesizer (Perkin-Elmer) or the like. The obtained oligonucleotides can be purified using a commercially available purification cartridge or the like, as desired.

### Real-time quantification and detection

The real-time P450 molecular species detection method by PCR using the primer pairs and probes of the present invention is described below in detail.

The real-time detection probes of the present invention comprise a reporter dye attached to one end, for example, the 5' end, and a quencher dye attached to the other end, for example, the 3' end. The reporter dye emits fluorescence, for example, upon irradiation with excitation light, and the quencher dye acts on the reporter dye to suppress the fluorescence emission when it is in close proximity to the reporter dye. Examples of reporter dyes include 6-carboxyfluorescein (FAM), tetrachloro-6-carboxyfluorescein (TET), 2,7-dimethoxy-4,5-dichloro-6-carboxyfluorescein (JOE), hexachloro-6-carboxyfluorescein (HEX), and the like. Examples of quencher dyes include 6-carboxytetramethylrhodamine (TAMRA) and the like.

A probe of the present invention can be prepared by attaching a reporter dye and a quencher dye to an oligonucleotide having the specific sequence. For example, a FAM molecule can be attached in the form of phosphoric acid ester to the phosphoric acid group at the 5' end of the probe, usually using several methylene chains as a linker. Further, a TAMRA molecule can be attached to the 3' end by an amide bond via the following structural unit.

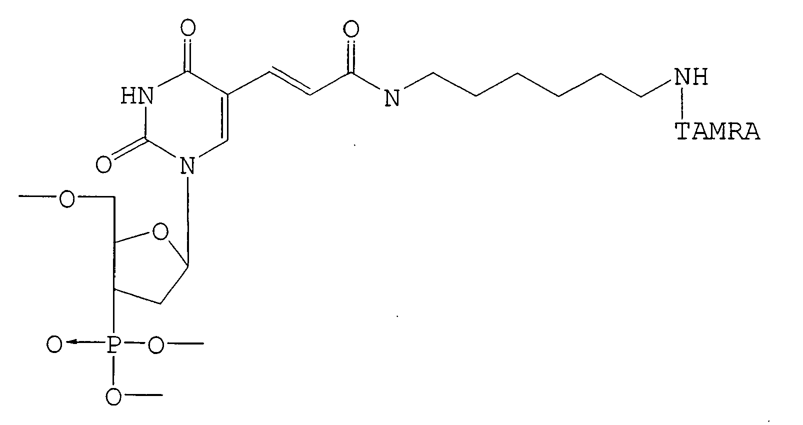

It is essential for the method of the present invention to use one or more of the primer pairs and probes of the present invention. Otherwise, the method can be conducted according to the known PCR (for example, Science, 230, 1350 (1985)) or RT-PCR (Genome Res., 6 (10), 986 (1996)), in particular, the real-time detection technique (for example, TaqMan PCR, ABI PRISMTM 7700 SEQUENCE DETECTION SYSTEM, Applied Biosystems, Ver1, June 1996).

The method of the present invention is especially useful for measuring the mRNA or cDNA of the target P450 molecular species, to determine the expression level of the P450 molecular species. In this case, a tissue expressing specific molecular species is cut out and the total RNA is extracted in a routine manner. Then, a cDNA complementary to the RNA is synthesized in a routine manner, followed by PCR using one or more of the primer pairs and probes of the present invention. Alternatively, after the extraction of total RNA in a routine manner, the RNA can be directly subjected to RT-PCR using one or more of the primers and probes of the present invention.

PCR and RT-PCR can be performed basically according to the known technique. The PCR or RT-PCR conditions can be selected according to the known technique and are similar to those employed therein. Specifically, the conditions shown in Examples given hereinafter can be preferably employed.

The detection can also be conducted basically according to the conventional technique, for example, by irradiating the PCR mixture with argon laser light and detecting the emitted fluorescence using a CCD camera.

The method of the present invention can easily and rapidly measure and detect the respective genes of P450 molecular species.

### Kit for detecting and quantifying P450 molecular species

The present invention also provides a kit for performing the above method. The kit comprises one or more sets of the above primer pairs and probes. The kit may further contain a known nucleic acid for use as a control, and a primer pair and probe for measuring the nucleic acid.

The method of the present invention can detect and quantify P450 molecular species individually. Moreover, the method of the present invention can rapidly and accurately quantify P450 molecular species. Accordingly, the application of the method of the present invention makes it possible to efficiently obtain fundamental data on the interaction and incompatibility of a new medicine with another medicine in humans.

### BEST MODE FOR CARRYING OUT THE INVENTION

Test Examples and Examples are given below to illustrate the present invention in further detail. Test Example 1 Preparation of calibration curves by the real-time detection technique

### (1) Test method

The real-time one-step RT-PCR technique employed in this test can inspect up to 96 samples at a time, by conducting up to 96 different reactions using 96 wells. Also, by this technique, RT and PCR can be performed in one tube.

The quantification is performed using two fluorescent dyes in close proximity, based on FRET produced when the wavelength regions of the fluorescence wavelength of one dye (reporter dye), and the excitation light wavelength of the other dye (quencher dye) overlap with each other. A probe (TaqMan probe), in which the two dyes causing FRET are attached to the ends, hybridizes with a cDNA derived from a particular P450 molecular species and amplified by PCR. The PCR extension reaction starts in this state, and the TaqMan probe is hydrolyzed by the 5'-3' endonuclease activity of TaqDNA polymerase, so that the reporter dye is released and the physical distance between the two dyes are increased. As a result, the fluorescence intensity of the reporter dye that has been suppressed by FRET is increased. Since the increase in the fluorescence intensity is proportional to the increase in amount of the PCR amplification product, the desired cDNA quantification can be achieved by measuring the increase in the fluorescence intensity after each PCR cycle.

In this test, FAM was attached as a reporter dye to the 5' end of the probe, and TAMRA was attached as a quencher dye to the 3' end. The attachment of these dyes and the preparation of the TaqMan probe were carried out by the techniques described in literature (Genome Res., 6 (10), 986 (1996)).

The oligonucleotides for use as the primers and probes were synthesized using an automatic DNA/RNA synthesizer (ABI), a dNTP substrate and prescribed reagents.

As sample RNAs, the following RNAs were isolated, purified and used in the form of total RNAs: CYP4B1 and CYP2F1 from the lung; CYP1B1 from the small intestine; CYP3A7 from a fetal liver pool; CYP4F8 from the prostate gland; CYP19 from the placenta; and CYP11B1, CYP11B2, and CYP17 from the adrenal gland. As sample RNAs of other molecular species, total RNAs purified from an adult liver pool was used. All these total RNAs were purchased from Clontech Laboratories, Inc.

The total RNAs were diluted with RNase-free water to 20 µg/mL, and then five-fold serially diluted with yeast tRNA (GIBCO) having a concentration of 50 µg/mL. Five microliters of each solution was used in the measurement.

RT-PCR was performed in a 50 µL/tube system, using TaqMan One-Step RT-PCR Master Mix Reagents Kit (PE Applied Biosystems) comprising a 300nM forward primer, a 900nM reverse primer, and a 200nM TaqMan probe, and ABI PRISM^{™}7700 Sequence Detection System (PE Applied Biosystems).

The PCR conditions were as follows: 1 cycle of 48°C for 30 minutes, 1 cycle of 95°C for 10 minutes, and 50 cycles each consisting of 95°C for 15 seconds and 60°C for 1 minute. The fluorescence intensity was measured after each cycle.

Table 1 shows the target molecular species and primer pairs and probes used in the above test. Table 2 presents the test results (calibration curves).

**Table 1**

| No. | Molecular species | Primer pair | | Probe |
|---|---|---|---|---|
| | | Forward primer | Reverse primer | |
| (1) | CYP1A1 | SEQ ID NO: 36 | SEQ ID NO: 37 | SEQ ID NO: 1 |
| (2) | CYP1A2 | SEQ ID NO: 38 | SEQ ID NO: 39 | SEQ ID NO: 2 |
| (3) | CYP1B1 | SEQ ID NO: 40 | SEQ ID NO: 41 | SEQ ID NO: 3 |
| (4) | CYP2A6/7 | SEQ ID NO: 42 | SEQ ID NO: 43 | SEQ ID NO: 4 |
| (5) | CYP2A6 | SEQ ID NO: 44 | SEQ ID NO: 45 | SEQ ID NO: 5 |
| (6) | CYP2A7 | SEQ ID NO: 46 | SEQ ID NO: 47 | SEQ ID NO: 6 |
| (7) | CYP2B6 | SEQ ID NO: 48 | SEQ ID NO: 49 | SEQ ID NO: 7 |
| (8) | CYP2C8 | SEQ ID NO: 50 | SEQ ID NO: 51 | SEQ ID NO: 8 |
| (9) | CYP2C9 | SEQ ID NO: 52 | SEQ ID NO: 53 | SEQ ID NO: 9 |
| (10) | CYP2C18 | SEQ ID NO: 54 | SEQ ID NO: 55 | SEQ ID NO: 10 |
| (11) | CYP2C19 | SEQ ID NO: 56 | SEQ ID NO: 57 | SEQ ID NO: 11 |
| (12) | CYP2D6 | SEQ ID NO: 58 | SEQ ID NO: 59 | SEQ ID NO: 12 |
| (13) | CYP2E1 | SEQ ID NO: 60 | SEQ ID NO: 61 | SEQ ID NO: 13 |
| (14) | CYP2F1 | SEQ ID NO: 62 | SEQ ID NO: 63 | SEQ ID NO: 14 |
| (15) | CYP2J2 | SEQ ID NO: 64 | SEQ ID NO: 65 | SEQ ID NO: 15 |
| (16) | CYP3A3/4 | SEQ ID NO: 66 | SEQ ID NO: 67 | SEQ ID NO: 16 |
| (17) | CYP3A4 | SEQ ID NO: 68 | SEQ ID NO: 69 | SEQ ID NO: 17 |
| (18) | CYP3A5 | SEQ ID NO: 70 | SEQ ID NO: 71 | SEQ ID NO: 18 |
| (19) | CYP3A7 | SEQ ID NO: 72 | SEQ ID NO: 73 | SEQ ID NO: 19 |
| (20) | CYP4A11 | SEQ ID NO: 74 | SEQ ID NO: 75 | SEQ ID NO: 20 |
| (21) | CYP4B1 | SEQ ID NO: 76 | SEQ ID NO: 77 | SEQ ID NO: 21 |
| (22) | CYP4F2 | SEQ ID NO: 78 | SEQ ID NO: 79 | SEQ ID NO: 22 |
| (23) | CYP4F3 | SEQ ID NO: 80 | SEQ ID NO: 81 | SEQ ID NO: 23 |
| (24) | CYP4F8 | SEQ ID NO: 82 | SEQ ID NO: 83 | SEQ ID NO: 24 |
| (25) | CYP11B1 | SEQ ID NO: 84 | SEQ ID NO: 85 | SEQ ID NO: 25 |
| (26) | CYP11B2 | SEQ ID NO: 86 | SEQ ID NO: 87 | SEQ ID NO: 26 |
| (27) | CYP17 | SEQ ID NO: 88 | SEQ ID NO: 89 | SEQ ID NO: 27 |
| (28) | CYP19 | SEQ ID NO: 90 | SEQ ID NO: 91 | SEQ ID NO: 28 |
| (29) | CYP27 | SEQ ID NO: 92 | SEQ ID NO: 93 | SEQ ID NO: 29 |
| (30) | CYP1B1 | SEQ ID NO: 94 | SEQ ID NO: 95 | SEQ ID NO: 30 |
| (31) | CYP2B6 | SEQ ID NO: 96 | SEQ ID NO: 97 | SEQ ID NO: 31 |
| (32) | CYP2C8 | SEQ ID NO: 98 | SEQ ID NO: 99 | SEQ ID NO: 32 |
| (33) | CYP2C9 | SEQ ID NO: 100 | SEQ ID NO: 101 | SEQ ID NO: 33 |
| (34) | CYP2C19 | SEQ ID NO: 102 | SEQ ID NO: 103 | SEQ ID NO: 34 |
| (35) | CYP4A11 | SEQ ID NO: 104 | SEQ ID NO: 105 | SEQ ID NO: 35 |

**Table 2**

| No. | Calibration curve | | Coefficient of correlation (r) | Limit of quantification (pg total RNA) |
|---|---|---|---|---|
| | Slope | Intercept | | |
| (1) | -3.20 | 40.68 | 1.00 | 32 |
| (2) | -3.19 | 34.63 | 1.00 | 1.28 |
| (3) | -5.31 | 47.40 | 0.99 | 4000 |
| (4) | -3.60 | 33.90 | 0.99 | 32 |
| (5) | -3.07 | 32.93 | 1.00 | 6.4 |
| (6) | -3.12 | 37.32 | 1.00 | 1.28 |
| (7) | -3.43 | 36.31 | 0.99 | 160 |
| (8) | -3.52 | 35.62 | 0.99 | 32 |
| (9) | -3.51 | 37.23 | 1.00 | 800 |
| (10) | -3.18 | 33.95 | 0.99 | 160 |
| (11) | -3.41 | 35.97 | 1.00 | 6.4 |
| (12) | -3.17 | 37.70 | 0.99 | 160 |
| (13) | -3.19 | 31.21 | 1.00 | 1.28 |
| (14) | -3.54 | 44.49 | 0.99 | 800 |
| (15) | -3.44 | 39.41 | 1.00 | 6.4 |
| (16) | -3.15 | 35.11 | 1.00 | 1.28 |
| (17) | -3.36 | 36.38 | 1.00 | 1.28 |
| (18) | -3.30 | 41.48 | 1.00 | 160 |
| (19) | -3.60 | 38.53 | 1.00 | 32 |
| (20) | -4.20 | 38.79 | 0.96 | 4000 |
| (21) | -3.22 | 36.23 | 1.00 | 160 |
| (22) | -2.95 | 35.24 | 1.00 | 1.28 |
| (23) | -3.49 | 37.21 | 0.99 | 6.4 |
| (24) | -3.41 | 37.97 | 1.00 | 6.4 |
| (25) | -3.26 | 36.35 | 1.00 | 1.28 |
| (26) | -3.02 | 36.02 | 1.00 | 32 |
| (27) | -3.18 | 30.32 | 1.00 | 1.28 |
| (28) | -3.21 | 31.07 | 1.00 | 1.28 |
| (29) | -3.39 | 37.65 | 1.00 | 6.4 |
| (30) | -3.34 | 43.42 | 1.00 | 160 |
| (31) | -3.33 | 36.84 | 1.00 | 1.28 |
| (32) | -3.29 | 34.53 | 1.00 | 1.28 |
| (33) | -3.29 | 34.43 | 1.00 | 1.28 |
| (34) | -3.52 | 41.10 | 1.00 | 6.4 |
| (35) | -3.28 | 35.08 | 1.00 | 1.28 |

In Table 2, the calibration curves prepared from RNA solutions that were five-fold serially diluted from 100000 pg total RNA/50 µL reaction mixture show that: CYP3A4 is quantifiable at a concentration as low as 1.28 pg total RNA/50 µL reaction mixture; and that the coefficients of correlation (r) of the calibration curves of other molecular species were 0.99 or higher when the limit of quantification was 1.28 pg to 4000 pg total RNA. However, the coefficient of correction of the calibration curve of CYP4A11 was 0.96.

### Test Example 2 Confirmation of P450 molecular species specificity of the primer-probe sets designed

### (1) Test method

This test was conducted as follows, to demonstrate that the method of the present invention can specifically distinguish P450 molecular species. The expression of P450 molecular species in tissues of the adrenal gland, liver, fetal liver, small intestine, kidney, lung, brain, prostate gland, testis, uterus and placenta was tested by the method of the present invention, in the same manner as in Test Example 1. The obtained results are compared with the results reported in literature.

GAPDH (glyceraldehyde-3-phosphate dehydrogenase) was used as the internal standard, and the test was performed in triplicate.

The total RNA pools of the tissues were purchased from Clontech Laboratories, Inc.

The total RNA concentration was 20000 pg total RNA/50 µL reaction mixture, for the tissues used for preparation of the calibration curves, and 100000 pg total RNA/50 µL reaction mixture, for other tissues.

Tables 3 and 4 present the results. The tables also show the characteristics (pooled number, age, sex, race, and cause of death) of the derivations of the total RNAs. In the tables, "ND" indicates less than the limit of quantification.

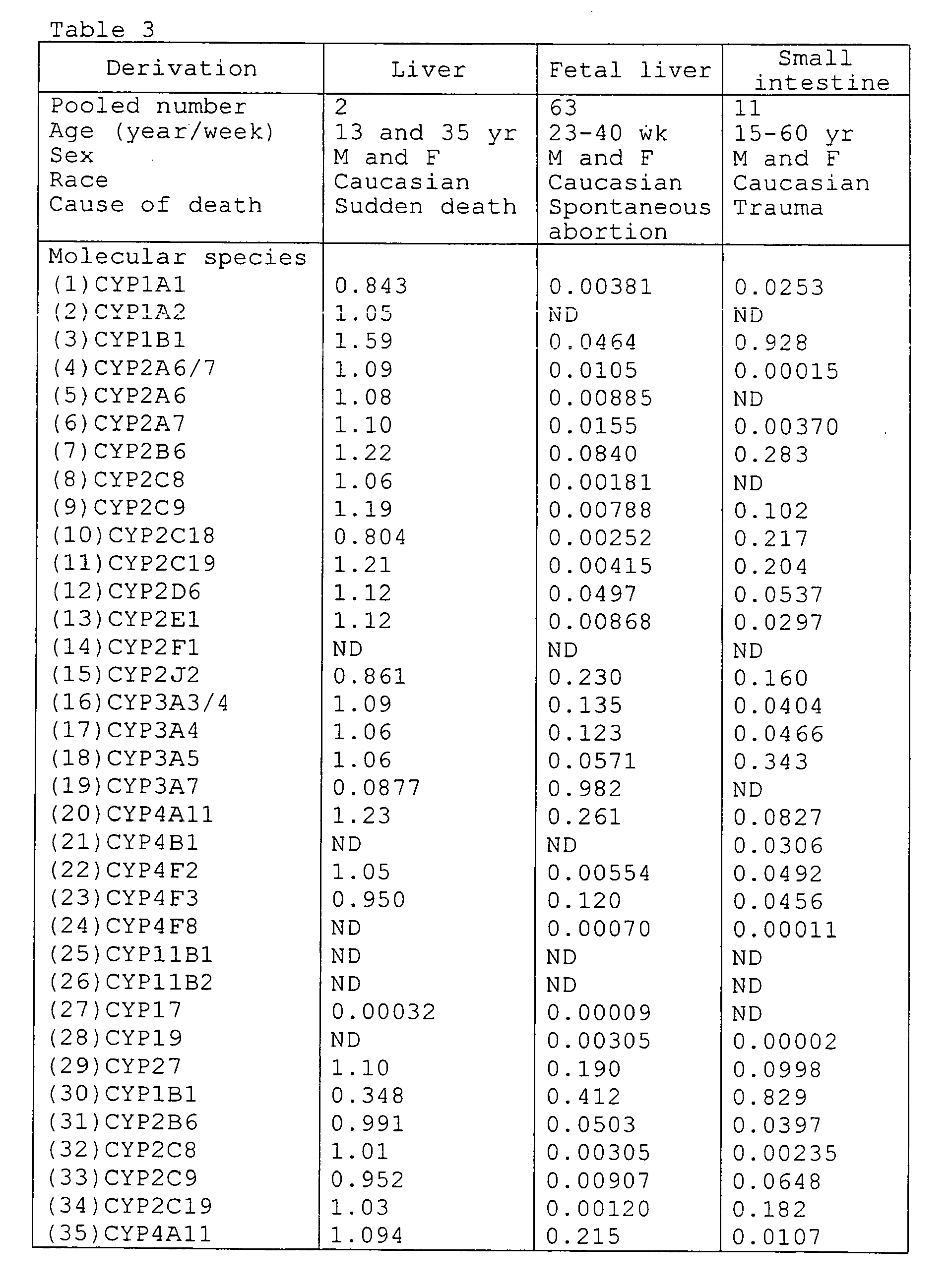

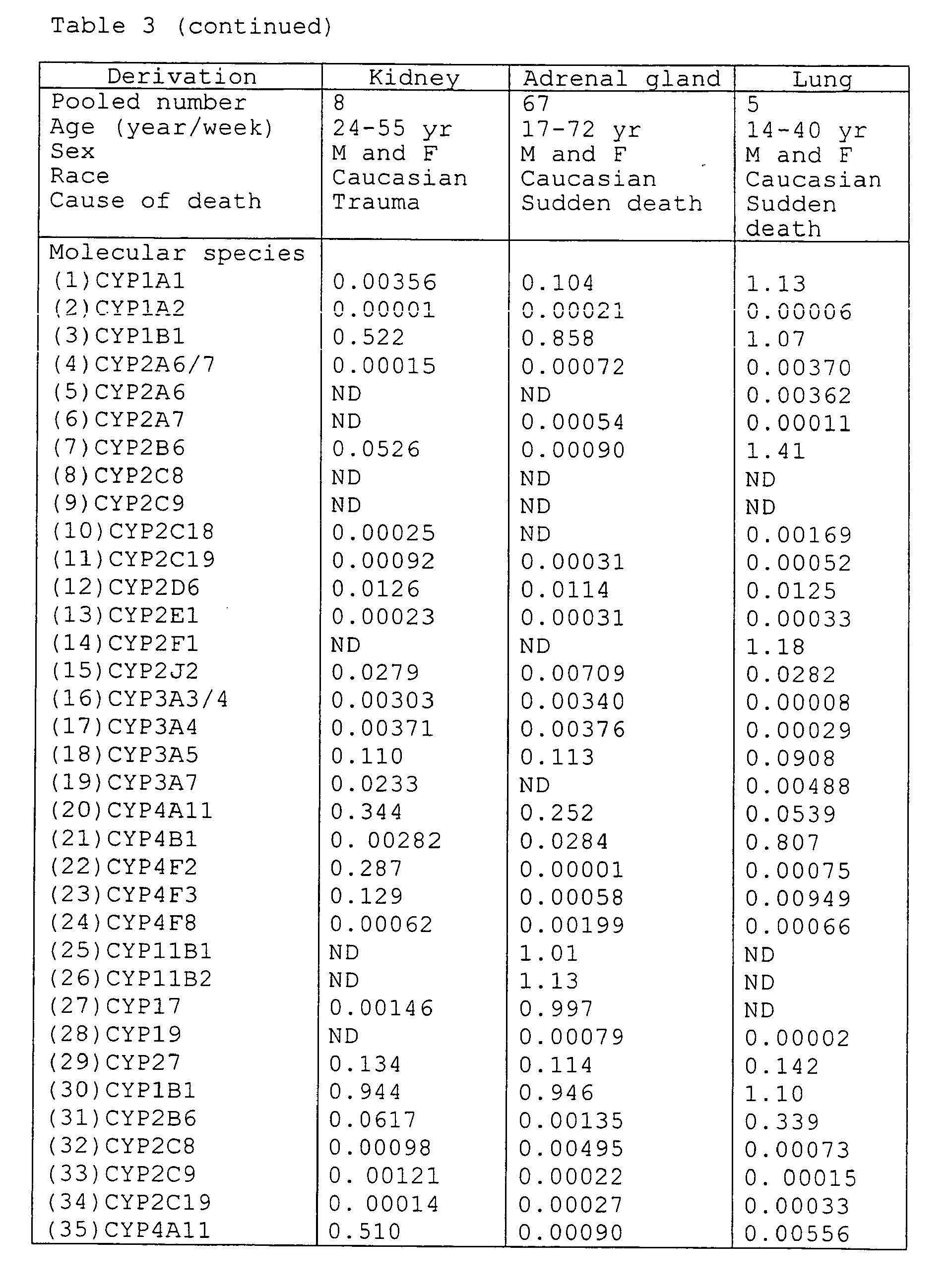

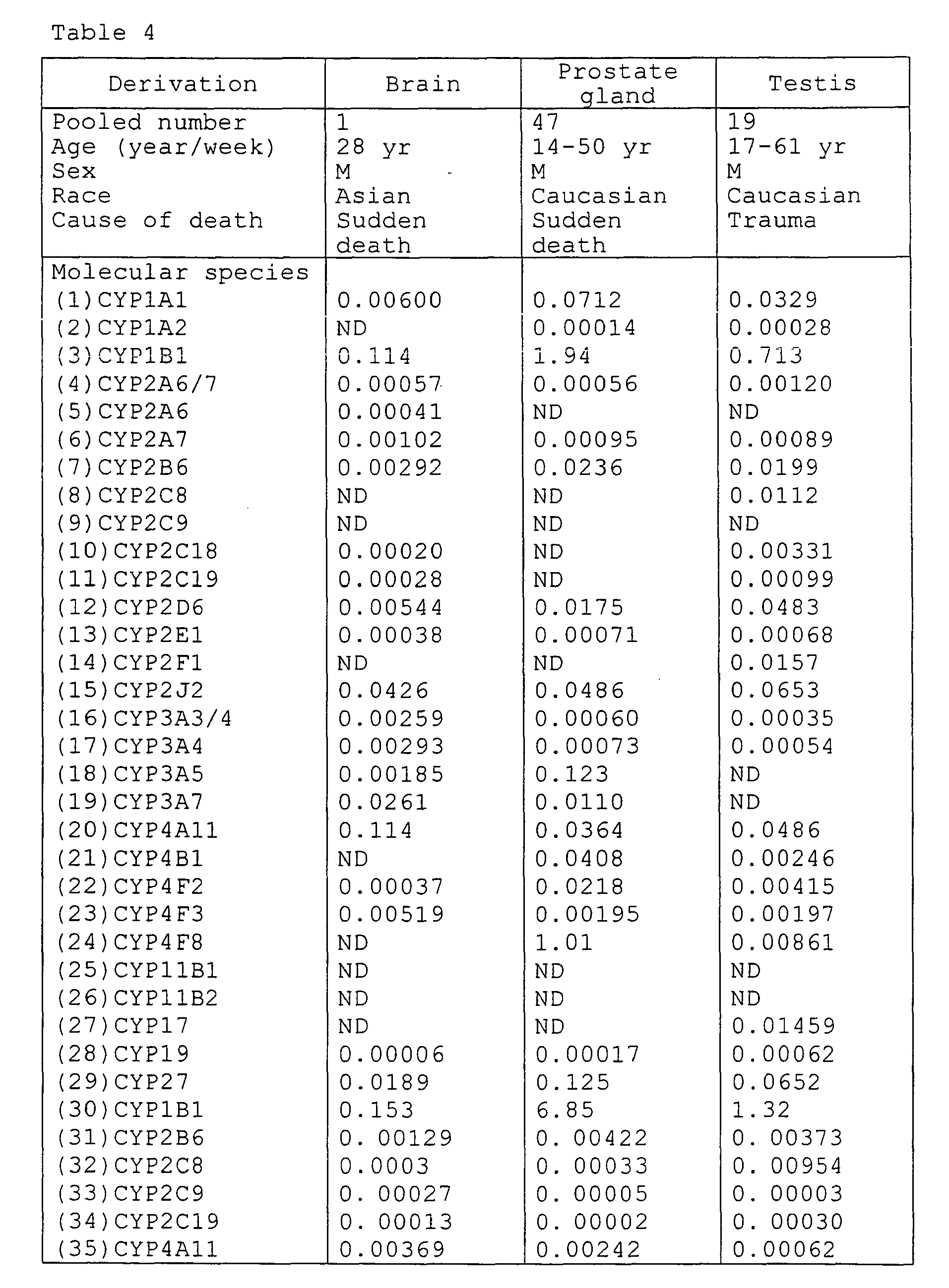

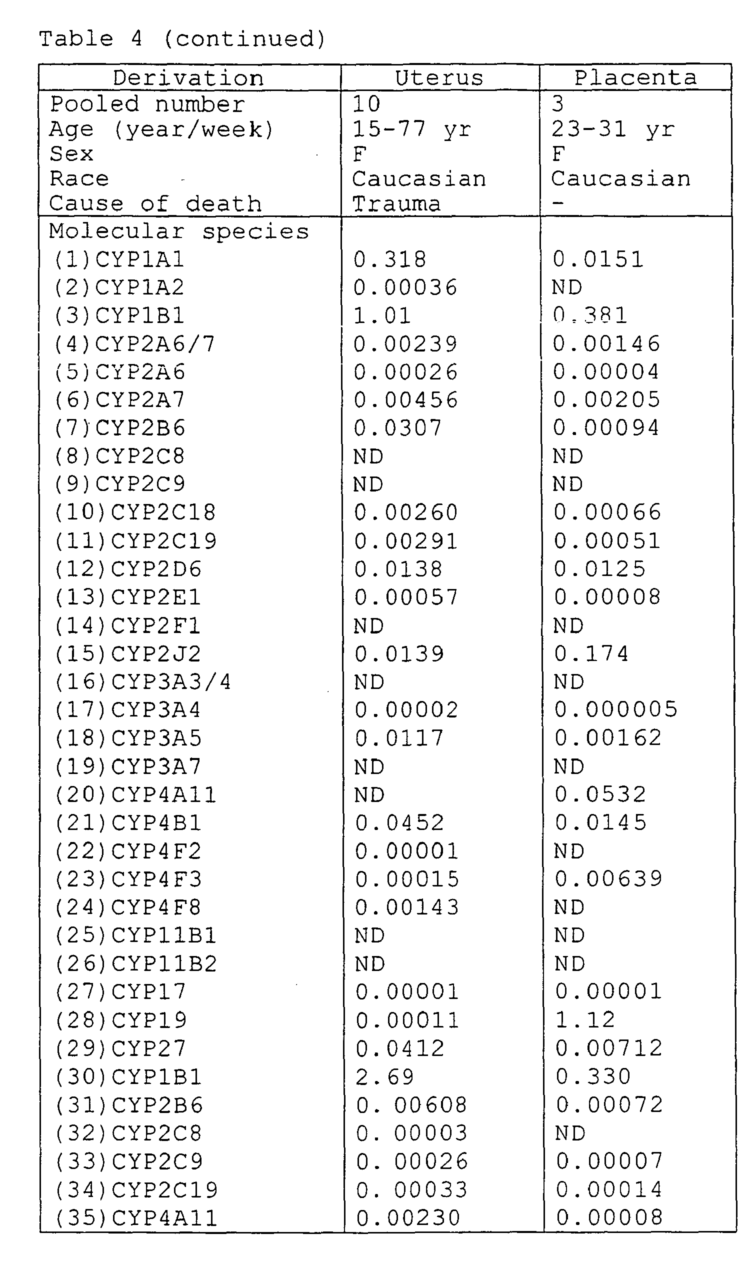

The results shown in the tables were compared with those obtained by the prior art techniques reported in the following Documents 1 to 5, and found to substantially correlate therewith. Further, molecular species that cannot be detected by the prior art techniques can be detected by the method of the present invention. The results achieved by the method of the present invention exhibit tissue-specific expression patterns that do not overlap with one another. Thus, it was demonstrated that the method of the present invention is capable of quantifying P450 molecular species individually.
Document 1: Drug Metabolism and Disposition 27, 804-809 (1999)
Document 2: Exp. Toxic. Pathol., 51, 412-417 (1999)
Document 3: Biochemical Pharmacology, 52, 379-383 (1996)
Document 4: Biochemical Pharmacology, 57, 1407-1413 (1999)
Document 5: Pharmacology & Therapeutics, 84, 429-445 (1999)

### INDUSTRIAL APPLICABILITY

The present invention provides a method for easily and rapidly detecting and quantifying human P450 molecular species individually in real time under the same PCR reaction conditions, and probes and primers for use in the method. The method of the present invention is useful in the clinical and medical fields.

## Claims

1. A kit for detecting and quantifying the CYP3A4 gene of the human cytochrome P450 molecular species, comprising a set of a primer pair of a forward primer comprising an oligonucleotide which hybridizes with the 825-854 region of the CYP3A4 gene and a reverse primer comprising an oligonucleotide which hybridizes with the 973-948 region of the CYP3A4 gene, and a probe comprising an oligonucleotide which hybridizes with the 946-918 region of the CYP3A4 gene.

2. A kit according to claim 1, wherein the probe further comprises a reporter dye and a quencher dye attached thereto.

3. A kit according to claim 2, which comprises a set of a primer pair comprising the sequences shown in SEQ ID Nos: 68 and 69 and a probe comprising the sequence shown in SEQ ID No: 17.

4. A kit according to claim 2, which comprises a set of a primer pair consisting of the sequences shown in SEQ ID Nos: 68 and 69 and a probe consisting of the sequence shown in SEQ ID No: 17.

5. A method of detecting and quantifying the CYP3A4 gene of the human cytochrome P450 molecular species, the method comprising the steps of :
(a) preparing a sample containing a human cytochrome P450 gene or genes,
(b) subjecting the sample to a polymerase chain reaction using a kit according to any one of claims 1 to 4 to amplify the CYP3A4 gene of the human cytochrome P450 molecular species in the sample; and
(c) detecting and measuring the probe or probes hydrolyzed during the polymerase chain reaction.

6. A method according to claim 5, wherein the hydrolyzed probe or probes are detected and quantified by detecting and measuring the fluorescence produced by irradiation with excitation light.
